(19) Europäisches Patentamt
European Patent Office
Office européen des brevets

(11) Publication number: 0 191 624 A2

# (12) EUROPEAN PATENT APPLICATION

(21) Application number: 86300909.8

(22) Date of filing: 11.02.86

(51) Int. Cl.⁴: C 07 F 1/12, A 61 K 31/52, A 61 K 31/28

(30) Priority: 12.02.85 US 700775

(43) Date of publication of application: 20.08.86 Bulletin 86/34

(84) Designated Contracting States: AT BE CH DE FR GB IT LI LU NL SE

(71) Applicant: ENGELHARD CORPORATION, 70 Wood Avenue South CN 770, Iselin New Jersey 08830 (US)

(72) Inventor: Hollis, Leslie S., 33 Guilford Lane, Mercerville New Jersey 08619 (US)
Inventor: Amundsen, Alan R., 12 Meadowbrook Drive, Somerville New Jersey 08876 (US)
Inventor: Stern, Eric W., 234 Oak Tree Road, Mountainside New Jersey 07092 (US)

(74) Representative: Fisher, Adrian John et al, Carpmaels & Ransford 43 Bloomsbury Square, London WC1A 2RA (GB)

## (54) Gold-purine antitumor agents.

(57) AuIII complexes containing purine derivatives have been prepared and show anti-tumor activity versus S180 ascites in mice. The gold-purine complexes have improved solubility and solution stability.

EP 0 191 624 A2

# GOLD-PURINE ANTITUMOR AGENTS

## Field of the Invention

This invention relates to coordination complexes of trivalent gold containing one or more ligands which are derivatives of purine. More particularly, this invention is concerned with coordination complexes of trivalent gold which show antitumor activity in mice and have excellent aqueous solubility and high aqueous stability.

## Background of the Invention

The development and subsequent commercialization of cisplatin (cis-diamminedichloroplatinum(II) during the 1970's has resulted in increased study of metal coordination compounds as medicinal agents. A large number of platinum compounds have been synthesized and tested as anticancer agents, along with somewhat lesser attention to other platinum group and non-platinum group transition metal compounds.

The use of gold compounds as antiinflammatory agents is well established. There are few published reports of gold compounds possessing antitumor activity. A series of trihalo-aminegold(III) compounds is the subject of an earlier Engelhard patent application, U.S. Serial Number 392,820.

Some gold compounds containing purines and their derivatives have been reported. These include $[Au(nucl)_2Cl_2]Cl$, $[Au(nucl)Cl_3]$, and $[Au(nucl-H^+)Cl_2]$, where nucl = inosine guanosine, triacetylinosine, and triacetylguanosine, (Journal of Inorganic Biochemistry, 14, 115-126, 1984). Gold compounds including a 1:1 and a 2:1 complex of gold with adenine, 1:1

complexes of gold with guanine, xanthine, and hypoxanthine, and gold complexes of AMP and GMP are also known. Oligomeric gold adenine complexes have also been suggested, (Biochemistry, 10, 3669, (1971)). None of these gold complexes have been structurally characterized, and antitumor activity has not been reported. The complex [Au(III)(5-diazauracil)$_2$Cl$_2$]Cl, although not a purine, was reported to possess antitumor activity in mice, C. Dragulescu et al, Int. Coord. Chem. Conf. XVI 1,99 (1974).

SUMMARY OF THE INVENTION

The gold compounds of the present invention include coordination complexes of trivalent gold containing 1 or more ligands which are derivatives of purine.

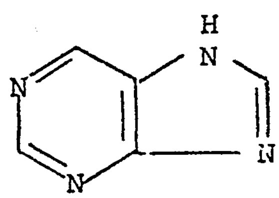

(I)

These derivatives may include purine itself, as well as such materials as guanine, adenine, xanthine, hypoxanthine, caffeine, theophylline, and their derivatives. The ligands may be either mono or bidentate and may be either in the neutral or protonated form.

The Au(III) purine complexes of this invention show antitumor activity.

BRIEF DESCRIPTION OF THE DRAWINGS

Figure 1 is a reproduction of the IR spectrum of a gold compound comprising the complex of gold and 7-chloroethyltheophylline ligand.

Figure 2 is a reproduction of the IR spectrum of a gold compound comprising a complex of gold with 7-(2,3-dihydroxypropyl) theophylline ligand.

## DETAILED DESCRIPTION OF THE INVENTION

The gold complexes of the present invention include compounds having the following general composition:

$AuL_i(HL)_jX_k$

where Au is in the +3 oxidation state; i and j are integers from 0-2 with the sum i+j having an integer value of 1 or 2; k is an integer having the value j+3.

L is a purine or derivative thereof and defined as a neutral molecule having the following structural formulas II, III, or IV.

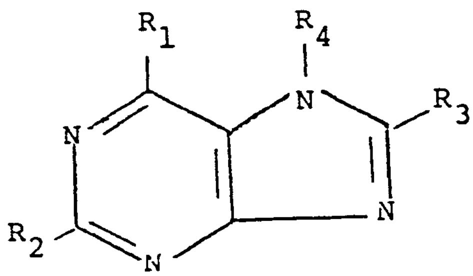

II

Where $R_1$ and $R_2$, and $R_3$ are chosen from: H; OH; $NH_2$; COOH; halo; linear, cyclic or branched $C_1$-$C_6$ alkyl; linear, cyclic, or branched $C_1$-$C_6$ alkoxy; linear, cyclic or branched $C_2$-$C_6$ alkylcarboxy; linear, cyclic or branched $C_2$-$C_6$ alkoxycarbonyl. The alkyl chains may in turn include one or more substitutents chosen from OH, $NH_2$, COOH, and halo.

$R_1$, $R_2$, and $R_3$ may not simultaneously be OH.

$R_4$ is chosen from H, or linear, cyclic or branched $C_1$-$C_6$ alkyl which is either unsubstituted or includes one or more substitutents chosen from OH, $NH_2$, COOH, or halo.

L may also be defined as:

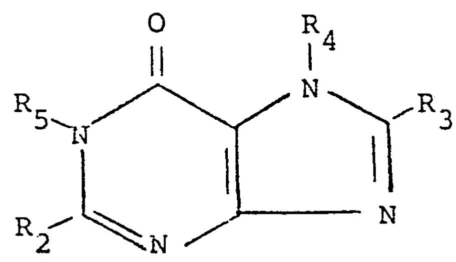

III

Where $R_2$, $R_3$, and $R_4$ are as defined above and $R_5$ is selected from the same group as $R_4$.

Additionally, L may be defined as:

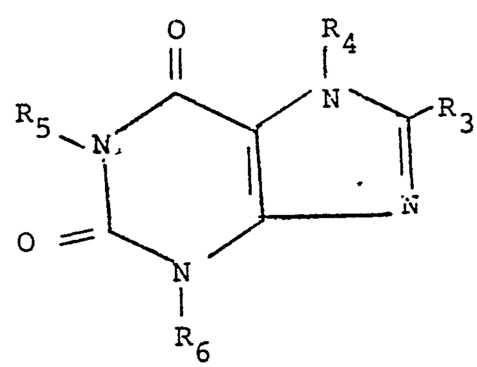

IV

Where $R_3$ and $R_4$ are as defined above and $R_5$ and $R_6$ are chosen from the same group as $R_4$.

X may be Cl or Br.

The complexes of this invention are useful in tumor chemotherapy having been found active against malignant tumors in animals as, for example, Sarcoma 180 ascites tumors in mammals such as mice. The anti-tumor effect exhibited by the subject complexes may also extend to other sarcomas and lymphoid leukemias and to such other tumors as the following: lymphosarcoma, myelocytic leukemia, malignant lymphoma, squamous cell carcinoma, adenocarcinoma, scirrhous carcinoma, malignant melanoma, seminoma, teratoma, choriocarcinoma, embryonalcarcinoma, cystadenocarcinoma, endometriocarcinoma or neuroblastoma and the like. In addition, said complexes may be useful as anti-viral, anti-inflammatory, anti-bacterial and antiparasitic agents.

The complexes of this invention may be administered parenterally or orally in admixture with a non-toxic pharmacologically acceptable inert carrier or diluent in any of the usual pharmaceutical forms. These include solid and liquid oral unit dosage forms such as tablets, capsules, powders, and suspensions or solutions and suspensions for subcutaneous, intramuscular, intravenous or intra-arterial injection. The term "unit dosage" refers to physically discrete units which may be administered in single or multiple dosages each containing a predetermined quantity of the active ingredient in association with the required diluent, carrier or vehicle. The quantity of active ingredient is the amount of complex needed to produce the desired therapeutic effect.

A typical unit dosage consists essentially of from about 10-450 mg. of active ingredient; however, the form in which said ingredient is administered and the frequency of administration is usually determinative of its concentration. Thus, for example, oral unit dosage forms containing 20-450 mg. of active ingredient may be administered one or more times per day depending upon the severity of the tumor which is sought to be treated and the condition of the host animal. By contrast, parenteral administration generally requires from about 10-100 mg. of the active ingredient per unit dosage administered as a daily dose or as a fraction thereof depending upon whether the regimen calls for administration once, twice, three or four times daily.

By contrast to the "unit dosage", the effective dose is that total dosage which is needed to achieve the desired anti-tumor effect. In general, this dosage lies within the range of from about 10-950 mg. of the active ingredient per kg. of body weight of the host animal (i.e., mg./kg.). A preferred concentration lies within the range of from about 30-450 mg./kg. For oral administration an effective dose of 50-950 mg./kg. has been found to be most suitable, whereas, in the case of parenteral administration it is usually advisable to employ from about 30-350 mg./kg. These dosages are well below the toxic or lethal dose and they may be varied over a wide range for the symptomatic adjustment of the dosage to the patient which is being treated.

The preferred compositions for oral administration are tablets in which the gold-purine complex is present in quantities of 5-375 mg. in a pharmaceutically acceptable orally ingestible solid carrier. If desired, the composition may also contain flavors, binders, lubricants and other excipients known in the art.

An alternative oral mode is the soft gelatin capsule. Such a composition may also contain from 10-375 mg. by weight of active ingredient dissolved or suspended in vegetable oil, peanut oil, alcohol or glycerine and the like.

A hard or dry-filled capsule may be prepared by admixing 25-200 mg. of active ingredient with suitable excipients such as lactose and magnesium stearate and putting the mixture into a preformed and suitably sized gelatin sheath.

The complexes of this invention may also be formulated as liquid solutions or suspensions or as a dry powder for addition to foods, drinking water, fruit juice or other potable liquids.

Tablets are prepared by mixing a complex of this invention, in suitably comminuted or powdered form, with a diluent or base such as starch, kaolin or dicalcium phosphate and the like. The resulting mixture can be granulated by wetting with a binder such as a syrup, starch (paste), acacia mucilage or solutions of cellulosic or polymeric materials, whereafter, the wetted mixture is forced through a screen. As an alternative to granulating, the powdered mixture can be run through a tablet machine and any slugs which are imperfect may be broken into granules. The granules are lubricated to prevent sticking to the tablet-forming dies via the addition of stearic acid, a stearate salt, talc or mineral oil and the lubricated mixture is then compressed into tablets. The complexes of this invention can also be combined with free flowing inert carriers and then be subjected to compression so as to form tablets without going through the granulating or slugging steps. A protective coating or sealing coat of shellac, sugar or polymeric material and a polished coating of wax can also be provided. Dyestuffs may be added to distinguish different unit dosages.

In this invention the term "pharmacologically acceptable inert carrier or diluent" denotes a non-toxic substance which, when mixed with the active ingredient, renders it more suitable for administration. Compositions intended for oral administration may include such carriers or diluents as lactose, corn starch, potato starch,

sodium carboxymethyl cellulose, ethyl cellulose, cellulose acetate, powdered gum tragacanth, gelatin, alginic acid, agar, stearic acid or the sodium, calcium and magnesium salts of stearic acid, sodium lauryl sulfate, polyvinylpyrrolidone, sodium citrate, calcium carbonate and di-calcium phosphate. Said compositions may also contain non-toxic adjuvants and modifiers such as dyes, buffering agents, preservatives, surfactants, emulsifiers, flavoring agents or biocides and the like.

The following emodiments illustrate the preparation of representative unit dosage forms.

Compressed Tablet

| | |
|---|---|
| Gold Complex with 7-chloroethyltheophylline Ligand (CET) | 200 mg. |
| Niacinamide | 50 mg. |
| Calcium Pantothenate | 20 mg. |
| Magnesium Sulfate | 50 mg. |
| Zinc Sulfate | 50 mg. |
| Magnesium Stearate | 10 mg. |
| | 380 mg. |

The gold-CET complex, niacinamide, calcium pantothenate, magnesium sulfate, zinc sulfate and magnesium stearate (5.0 Mg.) are mixed and compressed into slugs. The slugs are then broken into granules and sifted through an 8 mesh screen. Additional magnesium stearate (5.0 mg.) is added and the mixture is then compressed into tablets suitable for oral administration.

## Soft Gelatin Capsule

A soft elastic gelatin capsule is filled with the following ingredients:

| | |
|---|---|
| Gold Complex With 7-(2,3-dihydroxypropyl) Theophylline Ligand (DHPT) | 100 mg. |
| Wheat germ oil | 50 mg. |
| Sunflower seed oil | 100 mg. |
| | 250 mg. |

The gold-DHPT complex and wheat germ oil are mixed with sunflower seed oil and the resulting mixture is poured into gelatin capsules suitable for oral administration. An alternative embodiment provides for substituting sunflower seed oil and wheat germ oil with equal amounts of peanut oil to obtain an otherwise similar capsule which is also suitable for oral administration.

## Dry Filled Capsule

A hard dry-filled capsule may be prepared from the following ingredients:

| | |
|---|---|
| Gold-CET Complex | 200 mg. |
| Niacinamide | 50 mg. |
| Calcium Pantothenate | 10 mg. |
| Corn Starch | 150 mg. |
| | 410 mg. |

The gold-CET complex is reduced to a No. 60 powder. Niacinamide, calcium pantothenate and corn starch are passed through a No. 60 bolting cloth and these ingredients are added to the Gold-CET complex. This combination of ingredients is mixed for 10 minutes and then poured into a No. 3 size gelatin capsule.

Dry Powder

The following composition illustrates a representative dosage in dry powder form. In this embodiment the active ingredient is water soluble and it is combined with up to 60% by weight of a suitable flavoring agent. All quantities are in a weight-percent relationship.

| | |
|---|---|
| Gold-DHPT Complex | 25-90% |
| Flavoring Agent | 10-60% |
| Preservative | 0.1% |

The gold-DHPT complex, flavoring agent and preservative are thoroughly blended to afford a homogeneous dry powder which is readily soluble in water. The resulting formulation may be used as a food additive or it may be blended with other therapeutic agents to afford combination-type medicinals. Alternatively, said powder may be dissolved in a pharmacologically acceptable diluent to afford a solution which is suitable for oral administration.

Compositions intended for parenteral administration may include such diluents and carriers as water-miscible solvents as, for example, sesame oil, groundnut oil, aqueous propylene glycol and a solution of sodium riboflavin. Typical of said compositions are solutions which contain the active ingredient in sterile form. An embodiment illustrating a dosage form suitable for intravenous injection is set forth below.

Parenteral Solution

Injectable solutions can be formulated by mixing an ampoule of active ingredient with an ampoule of sterile diluent:

| | | |
|---|---|---|
| Ampoule: | Gold-CET Complex | 300 mg. |
| Ampoule: | Sterile water (Diluent for Injection) | 4 cc. |

The gold-CET complex and water are mixed thoroughly immediately prior to administration. If desired, one or more other active ingredients may be added to provide an injectable solution having enhanced therapeutic activity.

Examples 1-11, infra, illustrate the method by which the Au(III) purine complexes of this invention may be obtained and Example 12 describes the protocols used to evaluate their efficacy in mice. However, said examples are illustrative onl and this invention should not be construed as being limited thereto because it will be apparent to one of ordinary skill that obvious modifications may be effected and functionally equivalent regents may be substituted for those recited therei without departing from the spirit or scope of this invention.

## EXAMPLE 1

### Gold (7-Chloroethyltheophylline) Complex

1.97g of $HAuCl_4 \cdot 3H_2O$ (Engelhard) was dissolved in 50 ml of methanol and 2.43 g of 7-chloroethyltheophylline (Aldrich) was added to the solution with stirring. The resulting light yellow solution was warmed to 60°C for 2 hours and then cooled slowly to room temperature. After further cooling for 1 hour at ca. 4°C, the resulting orange-yellow crystals were removed by filtration and washed with methanol. The yield, after vacuum drying, was 2.9g (68.9%).

The elemental analysis data for the 7-chloroethyltheophylline (CET) complex suggests that the complex contains two theophylline molecules per gold atom. The best formulation, based on this data, is one that contains one CET as the HCl salt plus one water and one methanol of crystallization. This CET·HCl moiety may be either free, as CET·HCl of crystallization, or bound, as a second ligand coordinated to the gold atom. Anal. Calcd. for $AuC_{19}H_{29}N_8Cl_6O_6$: Au, 22.53; C, 26.07; H, 3.34; N, 12.80; Cl, 24.30. Found, Au, 22.46; C, 26.45; H, 3.39; N, 12.67; Cl 24.26.

## EXAMPLE 2

### Gold [7-(2,3-dihydroxypropyl)theophylline] Complex

3.94g of $HAuCl_4 \cdot 3H_2O$ (Engelhard) was dissolved in 200 ml of methanol and 2.55g of 7-(2,3-dihydroxypropyl) theophylline (Aldrich) was added to the solution with stirring. The resulting suspension was heated to 60°C for 3 hours and filtered to remove 2.5g of a yellow solid. A second crop, 1.7g, was obtained by filtration after cooling the above filtrate for 10 hours at ca. 5°C. The calculated total yield was 49%.

The 7-(2,3-dihydroxypropyl)theophylline (DHPT) complex appears to be a dihydrate containing both protonated deprotonated DHPT ligands. Anal. Calcd. for $AuC_{20}H_{33}N_8Cl_4O_{10}$: Au, 22.29; C, 27.16; H, 3.76; N, 12.67; Cl, 16.33. Found: Au, 22.00; C, 26.97; H, 3.26; N, 12.71; Cl, 16.33.

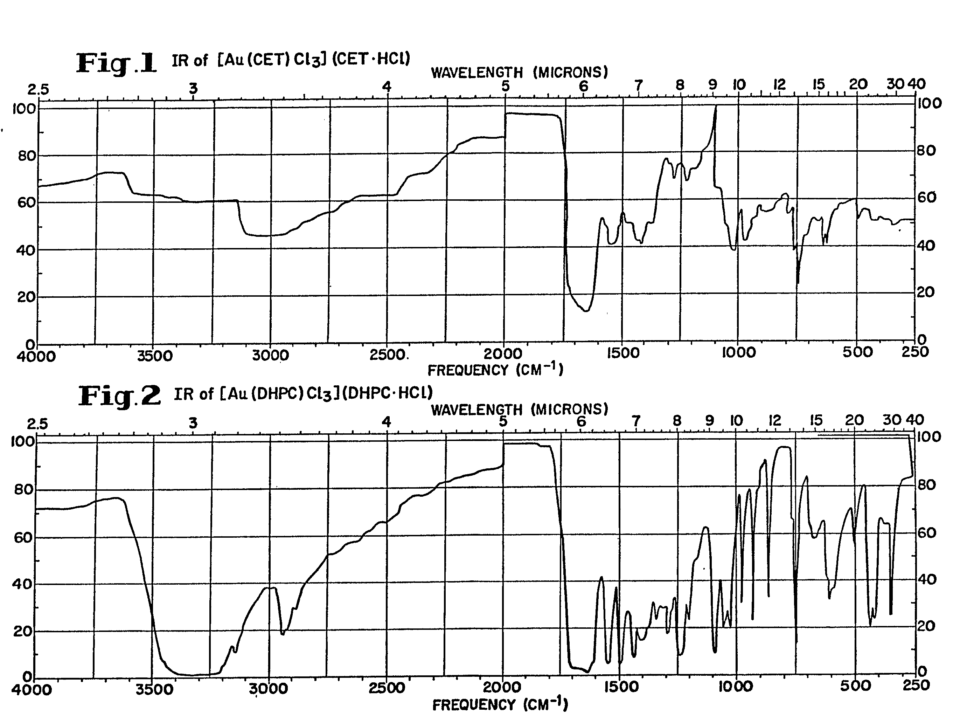

The IR spectra of both theophylline analogs are presented in Figures 1 and 2. While the IR spectra cannot be used to determine how the ligands are coordinated to the gold atom in these complexes, the spectra suggest that the two compounds are structurally similar.

## EXAMPLES 3 to 11

The following compounds are also prepared using the methods outlined in Examples 1 and 2:

| Example No. | L | X | i | j | k |
|---|---|---|---|---|---|
| 3 | guanine | Cl | 1 | 0 | 3 |
| 4 | purine | Br | 1 | 1 | 4 |
| 5 | caffeine | Cl | 2 | 0 | 3 |
| 6 | theobromine | Br | 2 | 0 | 3 |
| 7 | adenine | Cl | 1 | 1 | 4 |
| 8 | hypoxanthine | Br | 1 | 0 | 3 |
| 9 | 7(2-hydroxyethyl)-theophylline | Cl | 1 | 1 | 4 |
| 10 | 1(β-hydroxypropyl)-theobromine | Br | 2 | 0 | 3 |
| 11 | 1-theobromine-acetic acid | Cl | 1 | 1 | 4 |

EXAMPLE 12

Screening of Compounds for Anti-Tumor Activity Against S180a

The compounds were evaluated for anti-tumor activity against S180 ascites in female CFW Swiss mice by the following procedure:

CFW mice, averaging 20g, are immediately inspected and placed in newly prepared cages. On day zero the mice are inoculated with 0.2 ml. of a freshly prepared saline suspension (0.15 M NaCl) containing 1 x $10^7$ tumor cells/ml, or a total of 2 x $10^6$ cells. This inoculum is freshly prepared using "transfer" mice which have been injected with tumor cells the previous week. This inoculum is the end-product of a series of steps which involves (1) the removal of the cells from the peritoneal cavity of the sacrificed transfer mouse, (2) alternate centrifugation-washing (2-3 times with cold saline) to remove occasional blood and other undesirable components, and finally (3) dilution (1:3) of the packed cell volume with saline (the final centrifugation being carried out at 1,000 rpm for 2 min.). A cell count is made on a 2,000-fold dilution of this 1:3 suspension by means of a Coulter Counter. A final dilution to 1 x $10^7$ cells/ml. is made based on the averaged count. On day 1, solutions of the test compounds are prepared and the mice injected, with each mouse of a set of six being injected with the same test compound at the same dose level.

Also, on this day, two types of controls (6 mice/se are employed: (1) Normal (1 set): 0.5 ml. of the solvent medium used for the test compound, and (2) Positive control (1 set): a known anti-tumor agent (cis-[Pt$(NH_3)_2Cl_2$] in saline at 8 mg./kg.), used to test the response of the biological system.

The effectiveness of a compound is measured in te of the increase in life span (ILS) of the test animals relative to the controls (calculated from the day of tumor inoculation (day zero). In order to standardize the test data and permit intercomparisons to be made, the day of evaluation is arbitrarily taken as that day corresponding to twice the mean life-span (or average day of death) of the normal controls. This sets a practical upper limit of 100% on the ILS attainable. For calculation purposes, survivors on the day of evaluation are considered to have died on that day. The % ILS is formulated as:

$$\% \text{ ILS} = \left( \frac{\text{mean life-span of test mice}}{\text{mean life-span of control mice}} - 1 \right) \times 100$$

ILS values above 50% represent significant activity; those above 75% represent excellent activity.

Sarcoma 180 ascites antitumor screening data for the gold-purine complexes described in Examples 1 and 2 are given in Table 1.

TABLE 1

ANTITUMOR SCREENING RESULTS

| Compound | Dose (mg/kg) | %ILS | 30-Day Survivors | Control %ILS | Control 30-Day Survivors |
|---|---|---|---|---|---|
| [$AuCl_3$(7-chloroethyl-theophylline)](7-chloroethyltheophylline)·HCl (Example 1) | 5 | 8 | 0/6 | 96 | 4/5 |
| | 10 | -3 | 0/6 | | |
| | 20 | 14 | 0/6 | | |
| | 40 | 9 | 0/6 | | |
| | 80 | 79 | 3/6 | | |
| | 160 | 3 | 1/6 | | |
| [$AuCl_3$(7-(2,3-dihydroxy-propyl)theophylline)](7-(2,3-dihydroxypropyl)theophylline)·HCl (Example 2) | 10 | -13 | 0/6 | 80 | 3/6 |
| | 20 | -11 | 0/6 | | |
| | 40 | -3 | 0/6 | | |
| | 80 | 69 | 2/6 | | |
| | 160 | 34 | 1/6 | | |
| | 320 | -62 | 0/6 | | |

These gold compounds represent an advance over our previously reported gold compounds in the area of solubility an solution stability, coupled with no apparent loss of antitumor activity. Both the CET and DHPT complexes are soluble in water to the extent of at least 64 mg/5 ml and are stable, at least with respect to reduction, in solution for ca. 24 hours. Most of the previously prepared gold compounds were insoluble in water; those which could be solublized very quickly underwent reduction to Au(I) in solution.

These compounds are also of great interest because the contain ligands similar to the biologically important purines. It may be possible to utilize this to facilitate drug transport to DNA, their presumed site of action. The 7-chloroethyltheophylline ligand also contains a structural feature common in the alkylating agents, another important class of antitumor agents.

WHAT IS CLAIMED:

1. A gold compound comprising the following general composition:

$AuL_i(HL)_jX_k$

where Au is in the +3 oxidation state, i and j are integers from 0-2 with the sum i+j having an integer value of 1 or 2; k is an integer having the value j+3; and where L is selected from the group consisting of 7-chloroethyltheophylline or 7-(2,3-dihydroxypropyl)theophylline; and wherein X is chlorine or bromine.

2. The compound of claim 1 wherein j is 0.

3. The compound of claim 1 wherein j is at least 1.

4. A pharmaceutical composition comprising as an active ingredient a gold complex in combination with a non-toxic pharmacologically acceptable inert carrier or diluent, said gold complex having the following general composition:

$AuL_i(HL)_jX_k$

where Au is in the +3 oxidation state; i and j are integers from 0-2 with the sum i+j having an integer value of 1 or 2; k is an integer having the value j+3; L is a purine or derivative thereof and defined as a neutral molecule having the following structural formulas I, II, or III;

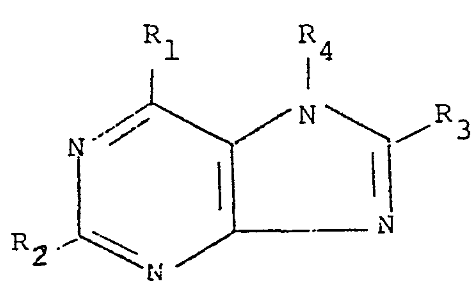

I

where $R_1$ and $R_2$, and $R_3$ are chosen from: H; OH: $NH_2$; COOH; halo; linear, cyclic or branched $C_1$-$C_6$ alkyl; linear, cyclic, or branched $C_1$-$C_6$ alkoxy; linear, cyclic or branched $C_2$-$C_6$ alkylcarboxy; linear, cyclic or branched $C_2$-$C_6$ alkoxycarbonyl; the alkyl chains being either unsubstituted or including one or more substitutents chosen from OH, $NH_2$, COOH, and halo; and $R_1$, $R_2$, and $R_3$ may not simultaneously be OH; $R_4$ is chosen from H, or linear, cyclic or branched $C_1$-$C_6$ alkyl which is either unsubstituted or includes one or more substitutents chosen frc OH, $NH_2$, COOH, or halo;

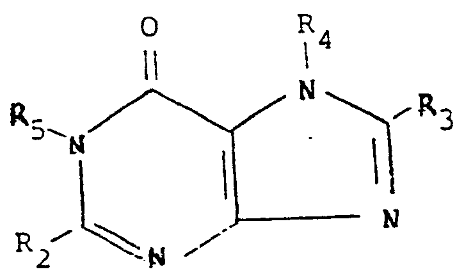

II

where $R_2$, $R_3$, and $R_4$ are as defined above and $R_5$ is selected from the same group as $R_4$;

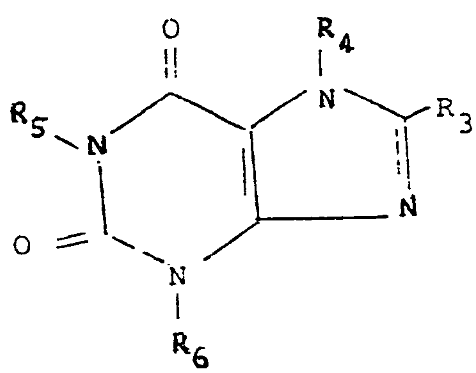

III

where $R_3$ and $R_4$ are as defined above and $R_5$ and $R_6$ are chosen from the same group as $R_4$; and X may be Cl or Br.

5. The composition of claim 4 wherein j is 0.

6. The composition of claim 4 wherein j is at least 1.

7. The composition of claim 4 wherein L is 7-chloroethyltheophylline

8. The composition of claim 4 wherein L is 7-(2,3-dihydroxypropyl)theophylline;

9. The composition of claim 4 wherein L is guanine.

10. The composition of claim 4 wherein L is purine.

11. The composition of claim 4 wherein L is caffeine.

12. The composition of claim 4 wherein L is theobromine.

13. The composition of claim 4 wherein L is adenine.

14. The composition of claim 4 wherein L is hypoxanthine.

15. The composition of claim 4 wherein L is 7(2-hydroxyethyl)-theophylline.

16. The composition of claim 4 wherein L is 1( -hydroxypropyl)-theobromine.

17. The composition of claim 4 wherein L is 1-theobromine-acetic acid.

18. A composition according to any of claims 4 to 17, adapted for parenteral administration.

19. A composition according to any of claims 4 to 17, adapted for oral administration.

20. A compound of the formula

$$AuL_i(HL)_jX_k$$

wherein Au is in the +3 oxidation state; and L, X, i, j and k are as defined in any of claims 4 to 17, for use in therapy.

21. The use of a compound of the formula

$$AuL_i(HL)_jX_k$$

wherein Au is the +3 oxidation state; and L, X, i, j and k are as defined in any of claims 4 to 17, in the manufacture of a medicament for treating animal malignant tumors.

22. A method for preparing a compound of the formula

$$AuL_i(HL)_jX_k$$

wherein Au is in the +3 oxidation state and L, X, i, j and k are as defined in any of claims 4 to 17, comprising reacting a comound of the formula

$$AuH_jX_k$$

with a compound of the formula L.

0191624

1/1

**Fig.1** IR of [Au(CET)$Cl_3$] (CET·HCl)

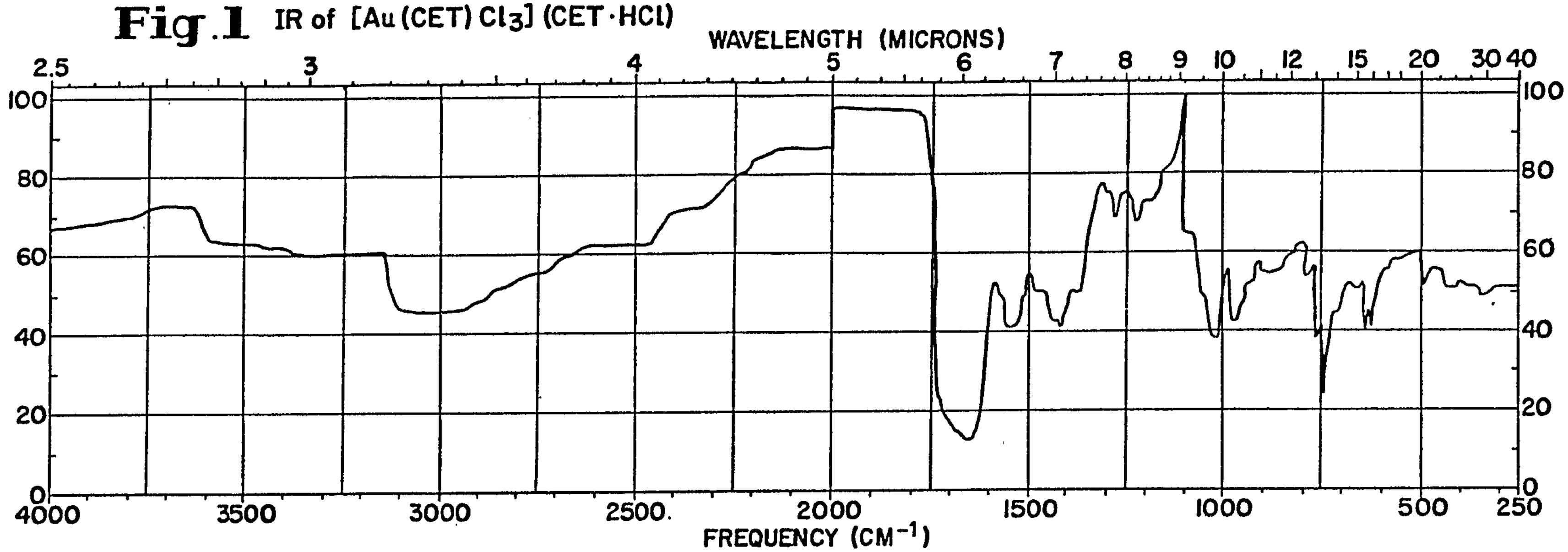


**Fig.2** IR of [Au(DHPC)$Cl_3$](DHPC·HCl)